# EUROPEAN PATENT APPLICATION

(11) **EP 0 550 007 A2**
(43) Date of publication of application: **07.07.1993**
(21) Application number: 92121863.2
(22) Date of filing: 23.12.1992
(51) Int. Cl.: C07D 487/04, C07D 519/00, A61K 31/40, A61K 31/435

(54) **Eseroline derivatives having anticholinesterase activity, a process for their preparation and pharmaceutical compositions containing them**

(30) Priority: 31.12.1991 IT MI913511
(71) Applicant: AESCULAPIUS FARMACEUTICI S.r.l., I-25125 Brescia (IT)
(72) Inventor: Puricelli, Laura, IT-25125 Brescia (IT)
(74) Representative: Gervasi, Gemma, Dr.

(57) **Abstract**

Eseroline derivatives having anticholinesterase activity of formula (I) 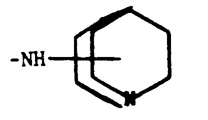
wherein Y is a substituent, as defined in the following Specification, HX a pharmaceutically acceptable organic or inorganic acid and n is the number of acid equivalents and is 0, 1/2, 1/3, 1 or 2;
a process for their preparation and pharmaceutical compositions, containing said derivatives for the treatment of diseases associated with cholinergic disorders.

## Description

### FIELD OF THE INVENTION

The present invention relates to eseroline derivatives having anticholinesterase activity, a process for their preparation and relative therapeutical compositions particularly suitable to improve memory.

### PRIOR ART

The anticholinesterase activity of physostigmine has been known since long, but this substance has the drawbacks of being particularly toxic, of having short action duration, and of exhibiting noxious peripheral effects, in particular at the expense of the digestive system.

### THE PRESENT INVENTION

The present invention regards eseroline derivatives having anticholinesterase activity of the general formula (I): 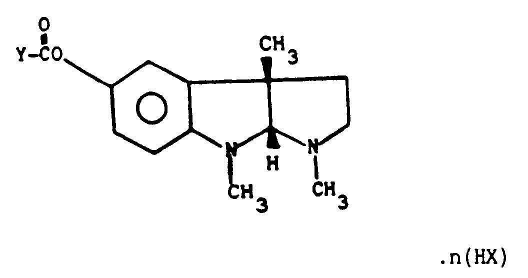
wherein Y is selected from meanings given in each of the following classes:
A) -NH-NR₁R₂ wherein R₁ and R₂ form together a saturated, heterocyclic ring of from 3 to 6 carbon atoms, optionally containing another heteroatom selected from N,O,S, and such heterocyclic ring being optionally substituted at its carbon atoms or at its second heteroatom, in case this is a nitrogen atom, with groups selected second from linear or branched C₁-C₁₀ alkyl radicals, optionally substituted on their turn with one or more hydroxy groups, or one or more carbon atoms of this heterocyclic ring optionally forming with one or more oxygen atoms, one or more 〉C=O groups;
B) -NH-R₃-T wherein R₃ is a C₁-C₁₀ linear or branched bivalent alkylene radical and T is a heterocyclic saturated radical containing from 3 to 4 carbon atoms and as heteroatoms one or more oxygen atoms.
C) 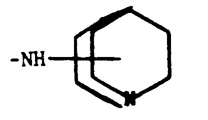
D) -NH-L wherein L is :
   i) a saturated, unsaturated or an aromatic type heterocyclic ring, containing at least a nitrogen atom and having from 3 to 6 carbon atoms and optionally containing other heteroatoms selected from N, O,S, said heterocyclic ring being optionally condensed to one or more aromatic rings, and being optionally substituted at the carbon atoms with alkyl, aldehyde, esters, hydroxy groups, or halogen atoms, one or more of said carbon atoms of this heterocyclic ring optionally forming with oxygen atoms 〉C=O groups,
      and wherein the nitrogen atoms of L are optionally substituted with one or more alkylene-hydroxy, aldehyde, or alkylene-esters groups; or
   ii) a saturated heterocyclic ring of from 1 to 2 oxygen atoms and of from 3 to 5 carbon atoms, being optionally condensed to one or more aromatic rings, or optionally substituted at the carbon atoms with one or more hydroxy groups or alkylene-hydroxy groups;
E) -NT'T'' wherein NT'T'' is a saturated heterocyclic ring of from 4 to 5 carbon atoms, substituted at the carbon atoms with one or more amidic groups, and said heterocyclic ring being optionally substituted at the remaining carbon atoms with one or more of hydroxy, alkylene-hydroxy and aldehyde groups.

HX is pharmaceutically acceptable organic or inorganic acid and n is the number of acid equivalents and is 0, 1/2, 1/3, 1 or 2.

The Applicant has in fact found that the duration of the inhibitory acetylcholinesterase activity of the compounds according to the present invention, is much longer than that of physostigmine, and moreover these compounds are much less toxic than the above mentioned active principle.

Therefore the present invention further relates to therapeutical compositions containing as the active principle one or more eseroline derivatives of the formula (I), in combination with suitable excipients and/or diluents.

Besides the present invention relates to the processes for preparing the compounds according to the present invention.

The first process according to the present invention allows to prepare the eseroline derivatives of formula (I) wherein Y is chosen among the meanings given in each of the above mentioned classes A, B, C and D, in particular it comprises the following steps:
a) reacting the eseroline of formula (II): 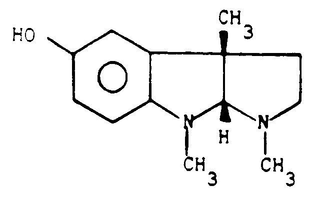 with the isocyanate of formula (III):

   G-N=C=O (III)

   wherein G has the following meanings :
   A') it is NR'₁R₂' wherein NR'₁R'₂ has the same meaning of -NR₁R₁R₂ with the only differences that when the heterocyclic ring contains a second nitrogen atom, this has been previously protected with a suitable protecting group and or in the case the heterocyclic ring brings one or more substituents consisting of alkylenehydroxy, these have been previously protected with suitable protecting groups;
   B') it is -R₃-T, wherein R₃ and T have the above mentioned meanings;
   C') it is 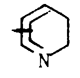
   D') it is L', wherein L' has the same meaning of L, with the only differences that when the heterocyclic ring contains one or more -NH- groups in its structure , these have been previously protected with a suitable protecting group, and/or in case the heterocyclic ring is substituted at the carbon atoms with alkylene-hydroxy or hydroxy groups, and/or at the nitrogen atoms with alkylene-hydroxy groups , these substituents have been previously protected with suitable protecting groups,
   thereby obtaining the intermediate (IV) 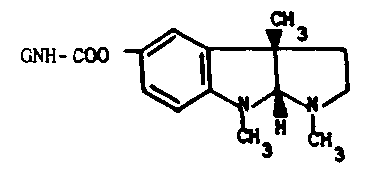
b) removing the above mentioned protecting groups, in case these are present, thereby obtaining the compounds of formula (I) having n=0;
c) reacting the intermediate obtained in step (a) or (b) with an equivalent amount of the pharmaceutically acceptable acid HX in an alcoholic solvent, thereby obtaining the eseroline derivative of formula (I) having n different from 0.

The second process according to the present invention permits to prepare all the compounds as defined by the general formula (I), and wherein therefore Y is chosen among the meanings given in each of the above mentioned classes A, B, C and D and E.

In particular it comprises the following steps:
a') reacting the eseroline of formula (II) under nitrogen atmosphere in an ethereal or in a halo-hydrocarbon solvent at room temperature, with the compound of formula (V):

   MCOM' (V)

   wherein M and M' have the following meanings :
   M = M'= imidazole;
   M = M' = 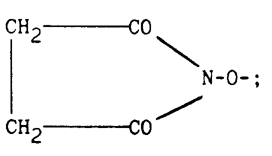 M =

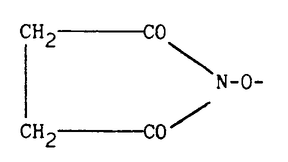

   M'= -O-CH₂-C(Cl)₃

   ;

   thereby obtaining the intermediate (VI) 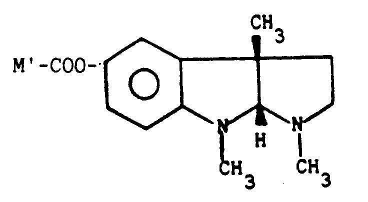
b') reacting the intermediate (VI) obtained in step (a) with the amine of formula (VII) :

   YH (VII)

   at a temperature comprised between -10 and 100°C in the same solvent used in step (a) in case the compound YH is soluble in this solvent otherwise reacting the above mentioned intermediate (VI) previously isolated from the reaction mixture of step (a) under the above cited operating conditions and with the above defined reactant (VII) in a dipolar aprotic solvent, thereby obtaining the eseroline derivative of formula (I) having n= 0;
c') treating the intermediate obtained in step (b) in an alcoholic solvent with equivalent amounts of the pharmaceutically acceptable acid HX, thereby obtaining the eseroline derivatives of formula (I), having n different from 0.

### DETAILED DESCRIPTION OF THE INVENTION

In the derivatives of formula (I) when HX is an organic acid it is preferably selected from tartaric acid, maleic acid, citric acid, when it is an inorganic acid, is preferably selected from hydrochloric, acid sulfuric acid, phosphoric acid.

When Y in the eseroline derivatives according to the present invention has the meanings given in class A it is preferably selected from: 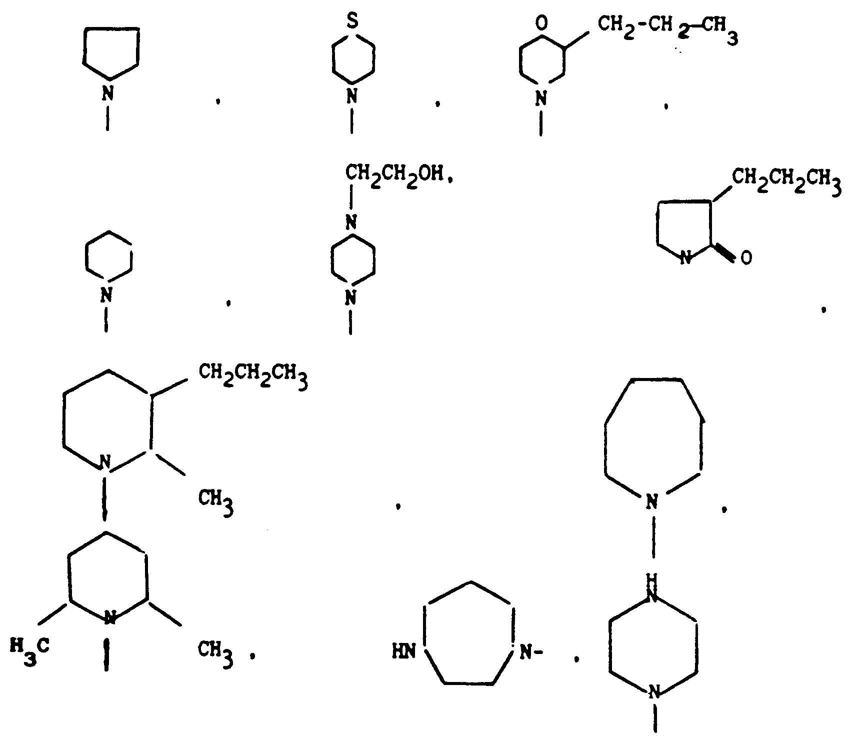
Whereas when Y has the meanings given in class B, R₃ is preferably -CH₂- and T is 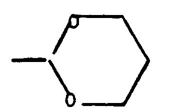
In the eseroline derivatives according to the present invention when Y has the meanings given in class C it is preferably : 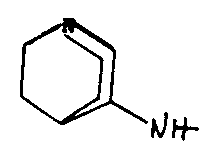
In the derivatives of formula (I) when Y has the meaning given in class D , L has the meaning given in subclass (i) and it is preferably 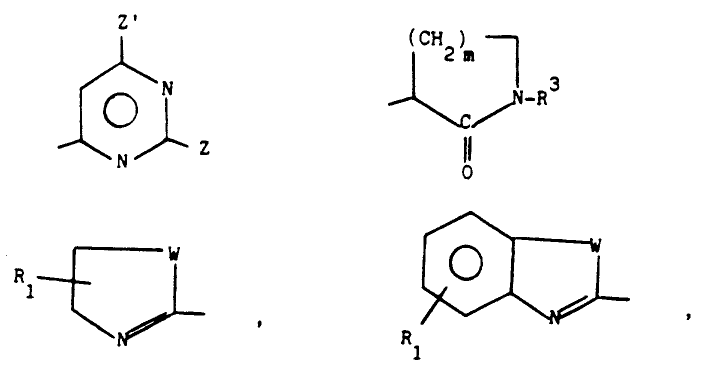
: wherein W = N,O,S; Z and Z' equal or different from eachother are OH or Cl; R₁ is selected from H,CHO, COOR₂,a halogen atom,OH, C₁-C₅ alkyl, R₂is C₁-C₅ alkyl R₃ is selected from H, CH₂-CH₂OH, CHO, CH₂COOR₂ wherein R₂ has the abovementioned meanings, and m is = 1, 2 or 3.

In the derivatives of formula (I) when Y has the meaning given in class D , L has the meaning given in subclass (ii) and it is preferably: 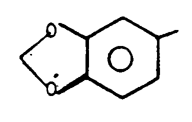
or glucosamine.

When Y in the eseroline derivatives has the meanings given in class E (-NT'T'') it is preferably: 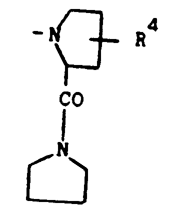
wherein R₄ is selected from: H, OH, CH₂OH, CHO.

As the compounds of the present invention exhibit inhibitory activity of the acetylcholinesterase enzyme, the relative therapeutical compositions besides being useful for the Alzheimer disease treatment they can also be advantageously utilized for the treatment of other diseases caused by cholinergic dysfunctions such as myasthenia gravis, glaucoma and other diseases of neuromuscular origin.

Moreover the therapeutical compositions according to the present invention may be advantageously used in the treatment of various memory dysfunctions, characterized by a cholinergic function reduction, occuring for example in the case of Alzheimer disease.

The Applicant has also found that some of the compounds, of the present invention of and particularly the compounds of formula (I) having Y is chosen among the meanings given in each of the above mentioned classes A, B and C, show also analgesic activity, therefore the present invention further relates to therapeutical compositions containing as the active ingredients the abovementioned eseroline derivatives of formula (I) wherein in particular the substituent Y has the meanings given in class A, B and C.

The therapeutical compositions according to the present invention contain preferably from 1 to 20 mg per unitary dose of one or more eseroline derivatives of formula (I) and are suitable to be both orally and parenterally administered.

Among the parenteral administrations, particularly preferred is the intravenous one.

The reaction described in step (a) in the first above mentioned process according to the present invention is preferably carried out at temperatures comprised between 20 and 60 °C, by using an apolar solvent such as benzene or toluene or a mixture of polar solvents, preferably tetrahydrofurane, and an apolar solvent, preferably toluene,optionally in the presence of catalytic amounts of sodium When G in the isocyanate derivative of formula (III) has the meanings given in class A,' the protecting group at the second nitrogen atom of the heterocyclic ring NR₁'R'₂ is preferably the carbobenzyloxy (CBZO), while the protecting group of the hydroxyl function of the hydroxyalkylene substituent on the heterocyclic ring is the benzyloxy group (BZO).

Analogously we may say that when G in the isocyanate derivative of formula (III) has the meanings given in class C' the protecting groups in the heterocyclic ring L' at the nitrogen atom(s) are preferably carbobenzyloxy (CBZO) groups , and/or in the same heterocyclic ring L' the protecting groups of the hydroxyl and of the hydroxyalkylene substituents at the carbon atoms and of the hydroxyalkylene substituents at the Nitrogen atom(s) are preferably benzyloxy (BZO) groups.

The removal of the above mentioned protecting groups (step b) of the process according to the present invention occurs by carrying out a hydrogenation at atmospheric pressure and at room temperature using as the catalyst Pd/carbon or PtO₂ in the presence of a mixture of an aromatic solvent and an alcohol, preferably benzene or toluene in a volumetric ratio 1:1.

The polar solvent in the step (c) of the process object of the present invention is preferably absolute ethanol.

The isocyanate of formula (III) is prepared by a process comprising the following steps:
a'') the primary amine of formula (IX):

   GNH₂ (X)

   wherein G has the above mentioned meanings is reacted with a phosgene excess in ethyl acetate at room temperature and successively heated to 35-40°C.
b'') the product obtained in the preceding step is recovered by crystallization from carbon tetrachloride.

In the second process according to the present invention, the solvent utilized in steps (a') and (b')is preferably selected from anhydrous tetrahydrofuran and methylene chloride.

When the compounds YH is insoluble in the solvent of step (a'), then said solvent is evaporated from the reaction mixture containing the intermediate (VI) and in step (b') dimethylformamide is preferably used as the solvent. The alcoholic solvent in step (c') is preferably absolute ethanol.

The following examples are reported for illustrative but not limitative purposes.

### Example 1

[Preparation of (3-aS-cis)-1,2,3,3a,8,8a-hexahydro 1,3a,8-trimethylpyrrole [2,3-B]-indole 5-ol-1-pyrrolidinyl-carbamate ester;

Derivative of formula (I) wherein 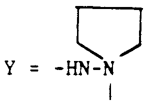
n=0 (ESE 001)].

2.183 g eseroline are placed into a flask fitted with a stirrer and a reflux condenser, 1.12 g 1-pyrrolidinyl isocyanate (derivative of formula (III) wherein 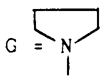
dissolved in 200 ml benzene are added. The mixture thus obtained is left under slow stirring for 3 hours.

At the end of the reaction the benzene is evaporated and 80 ml petroleum ether are added to the residue, followed by 40 ml 0.1 M hydrochloric acid.

The solvent is removed and the aqueous solution is extracted with ethyl ether after neutralization of the acid solution with sodium bicarbonate, the ethereal extracts are collected and dried on anhydrous sodium sulfate.

The solvent is evaporated and the residue is dissolved in 10 ml of a mixture chloroform/methanol (98/2).

The solution is eluted on a chromatographic silica gel column, utilizing as the eluent a mixture of chloroform/methanol in the same above mentioned volumetric ratio.

The solvent of the fraction containing ES001 is evaporated by the aid of vacuum.

The residue is dissolved in 10 ml benzene, and by adding heptane a pure white crystalline precipitate of ESE001 is obtained (2.47 g) yield about 75 %.

The spectral analyses confirm its structure.

| Elemental Analysis | C | H | N |
|---|---|---|---|
| calculated (%) | 66.252 | 8.193 | 16.265 |
| found (%) | 66.3 | 8.2 | 16.3 |

Molecular weight 344.456

### Example 2

[Preparation of (3-aS-cis)-1,2,3,3a,8,8a-hexahydro 1,3a,8-trimethylpyrrole [2,3-B]-indole 5-ol-1-piperazinyl-carbamate ester; Derivative of formula (I) wherein 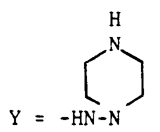
n=0 (ESE002)].

Following the same working conditions as in the example 1 eseroline is reacted with carbobenzyloxy piperazinyl-isocyanate (derivative of formula (III) wherein G is:

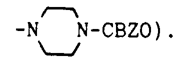

At the end of the reaction, benzene is evaporated and 80 ml of petroleum ether are added to the residue, followed by 40 ml of 0.1 M hydrochloric acid.

The solvent is eliminated and the aqueous solution is extracted with diethyl ether after neutralization of the acid solution with sodium bicarbonate, the ethereal extracts are then collected and dried on anhydrous sodium sulfate.

The solvent is evaporated, the residue is dissolved in 10 ml of a mixture chloroform/methanol (98/2).

The solution is separated by chromatography on silica gel column by using as the eluent the chloroform/methanol mixture above mentioned.

The fraction containing the desired product is evaporated with the aid of vacuum.

The residue is dissolved into 20 ml of ethanol/toluene (1/1) mixture, then a hydrogenate is carried out at atmospheric pressure and at room temperature using as catalyst 10% Pd/C.

At the end of the reduction the filtered solution is evaporated to dryness by the aid of vacuum. The residue is dissolved in 10 ml of benzene, after addition od heptane a white crystalline precipitate of ESE002 is obtained.

The spectral and elemental analyses confirm its structure.

### Example 3

[Preparation of (3-aS-cis)-1,2,3,3a,8,8a-hexahydro 1,3a,8-trimethylpyrrole [2,3-B]-indole 5-ol-1-morpholinyl-carbamate ester; Derivative of formula (I) wherein 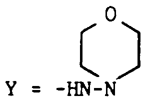
n=0 (ESE003)] Following the same working conditions as in the example 1 the derivative ESE003 is prepared starting from eseroline and morpholinylisocyanate (derivative (III) wherein

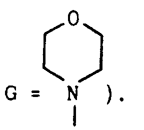

The spectral and elemental analyses confirm its structure.

### Example 4

[Preparation of (3-aS-cis)-1,2,3,3a,8,8a-hexahydro 1,3a,8-trimethylpyrrole [2,3-B]-indole 5-ol-1-thio-morpholinyl-carbamate ester; Derivative of formula (I) wherein 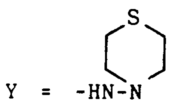
n=0 (ESE0004)].

Following the same working conditions as in the example 1 the derivative ESE004 is prepared starting from eseroline and thiomorpholinylisocyanate (derivative (III) wherein

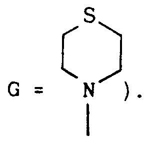

The spectral and elemental analyses confirm its structure.

### Example 5

[Preparation of (3-aS-cis)-1,2,3,3a,8,8a- hexahydro 1,3a,8-trimethylpyrrole [2,3-B]-indole 5-ol-1-[4(2-hydroxyethyl-piperazinyl)carbamate ester.

Derivative of formula (I) wherein 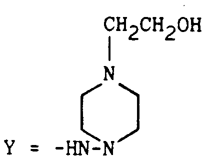
n=0 (ESE005)]. Following the same working conditions as in the example 2 the derivative ESE005 is obtained starting from eseroline and from 1[4-(2-benzyloxyethyl)-piperazinyl]-isocyanate. (derivative of formula (III) wherein G is 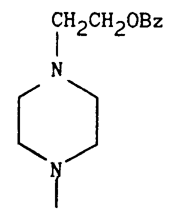
The spectral and elemental analyses confirm its structure.

### Example 6

[Preparation of (3-aS-cis)-1,2,3,3a,8,8a- hexahydro 1,3a,8-trimethylpyrrole [2,3-B]-indole 5-ol-homopiperazinylcarbamate ester. (Derivative of formula (I) wherein 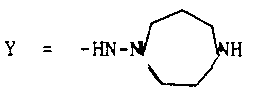
n=0 ESE006).

Following the same working conditions as in the example 2 the derivative ESE006 is prepared starting from eseroline and from 4-carbobenzyloxy-homopiperazinylisocyanate.
(derivative (III) wherein

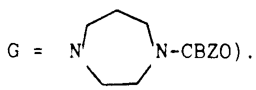

The spectral and elemental analyses confirm its structure.

### Example 7

[Preparation of (3-aS-cis)-1,2,3,3a,8,8a- hexahydro 1,3a,8-trimethylpyrrole [2,3-B]-indole 5-ol- 1-(2,6 -dimethyl) piperidine carbamate ester. derivative of formula (I) wherein Y is 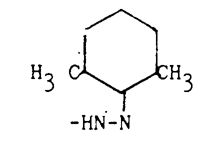
n=0 (ESE007)].

Following the same working conditions of the example 1 the compound ESE007 is prepared starting from eseroline and from 2,3 dimethylpiperidinylisocyanate (derivative of formula (III) wherein G = 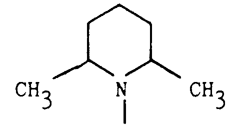
The spectral and elemental analyses confirm its structure.

### Example 8

[Preparation of (3-aS-cis)-1,2,3,3a,8,8a- hexahydro 1,3a,8-trimethylpyrrole [2,3-B]-indole 5-ol-2-methyl-1,3 dioxolane carbamate ester.

Derivative of formula (I) wherein 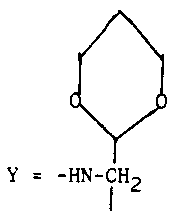
n=0 (ESE008)].

Following the same working conditions of the example 1 the compound ESE008 is prepared starting from eseroline and 2-methylen-1,3 dioxolan-isocyanate.
(derivative of formula (III) wherein 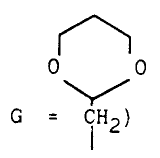
The spectral and elemental analyses confirm its structure.

### Example 9

[Preparation of (3-aS-cis)-1,2,3,3a,8,8a-hexahydro 1,3a,8-trimethylpyrrole [2,3-B]-indole 5-ol-4-(3-propyl)-morpholinylcarbamate ester.

Derivative of formula (I) wherein 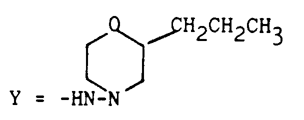
n=0 (ESE009)] Following the same working conditions of the example 1 the compound ESE009 is prepared starting from eseroline and 4-(3-propyl)-morpholinyl-isocyanate.
(derivative of formula (III) wherein 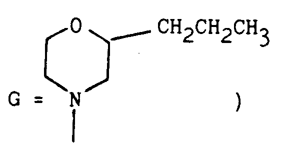
The spectral and elemental analyses confirm its structure.

### Example 10

[Preparation of (3-aS-cis)-1,2,3,3a,8,8a- hexahydro 1,3a,8-trimethylpyrrole [2,3-B]-indole 5-ol- 1-(3-propyl)-2-pipecolinylcarbamate ester.

Derivative of formula (I) wherein 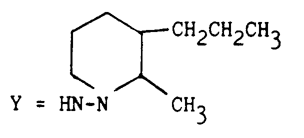
n=0 (ESE010)].

Following the same working conditions as in the example 1 the compound ESE010 is prepared starting from eseroline and 1-(3-propyl) pipecolinyl-isocyanate.
(derivative of formula (III) wherein 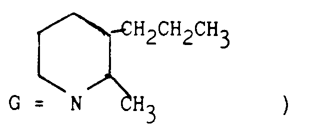
The spectral and elemental analyses confirm the structure.

### Example 11

[Preparation of (3-aS-cis)-1,2,3,3a,8,8a- hexahydro 1,3a,8-trimethylpyrrole [2,3-B]-indole 5-ol-1-(3-propyl)-2 pyrrolidone-carbamate ester.

Derivative of formula (I) wherein 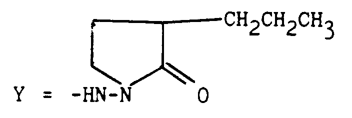
n=0 (ESE011)].

Following the same working conditions of the example 1 the compound of formula ESE011 is prepared starting from eseroline and 1-(3-propyl) 2-pyrrolidone isocyanate.
(Derivative of formula (III) wherein 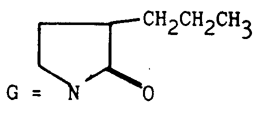
The spectral and elemental analyses confirm its structure.

### Example 12

[Preparation of (3-aS-cis)-1,2,3,3a,8,8a-hexahydro 1,3a,8-trimethylpyrrole [2,3-B]-indole 5-ol-3 quinuclidyl-carbamate ester. derivative of formula (I) wherein 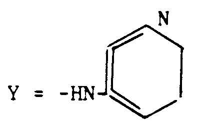
n=0 (ESE012)].

Following the same working conditions of the example 1 the compound ESE012 is obtained starting from eseroline and 3 quinuclidylisocyanate.
(derivative of formula (III) wherein 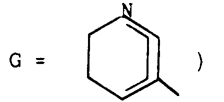
The spectral and elemental analyses confirm the structure.

### Example 13

[Preparation of piperidine isocyanate (derivative of formula (III) wherein 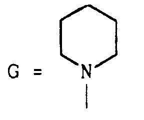
is used as reagent as in the example 1)].

100 ml of ethyl acetate saturated with phosgene are transferred into a 1 l flask.

10.07 g 1-amino piperidine dissolved in 200 ml ethyl acetate are added in 3 hours under slow stirring into the solution saturated with phosgene.

While the 1-aminopiperidine solution is added, phosgene is introduced into the reaction so that it is always maintained in excess.

At the end of the reaction, the solution is mildly heated in order to dissolve the 1-aminopiperidine hydrochlorate.

The ethyl acetate is distilled and the obtained residue of brown color is treated with 50 ml carbon tetrachloride, a solution containing a precipitate consisting of disubstituted urea is obtained, which is separated by filtration. 2/3 of the carbon tetrachloride are distilled.

In the concentrated solution thus obtained a crystalline precipitate is observed, which is filtered and at a first analysis it results to be 1-piperidine isocyanate.

By further concentrating the mother liquors, other 1-piperidine isocyanate is obtained. The collected products are crystallized from carbon tetrachloride; 7.45 g of pure product are obtained (yield 59 %).

The spectral analyses confirm the structure.

| Elemental analysis | C | H | N |
|---|---|---|---|
| calculated (%) | 57.124 | 7.990 | 22.20 |
| found (%) | 57.2 | 8.1 | 22.10 |

Molecular weight: 126.05

### Example 14

[Preparation of citrate of (3-aS-cis)-1,2,3,3a,8,8a-hexahydro 1,3a,8-trimethylpyrrole [2,3-B]-indole 5-ol-1-piperazine carbamate ester.

Derivative of formula (I) wherein 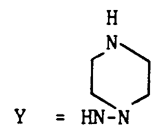
n = 1 HX = citric acid).

0.1 moles ESE002 obtained from example 2 and 0.1 moles citric acid are dissolved in 100 ml absolute ethanol. After dissolution the solvent is evaporated with the help of vacuum.

The elemental and spectral analyses confirm its structure.

### Example 15

Preparation of eseroline R-caprolactam carbamate (MGT1) - Derivative of formula (I) wherein Y = L-NH
and wherein L = 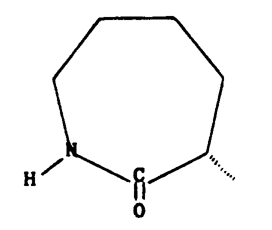
6.55 g (30 mmol) eseroline in 50 ml methylene chloride are introduced into a 250 ml flask fitted with a magnetic stirrer, a nitrogen inlet tube and a reflux condenser provided with a calcium chloride valve. The oxygen is eliminated by a continuous nitrogen flow for 45 minutes, therefore 5.77 g (35.6 mmol) N,N'- carbonyl diimidazole are added. The mixture is reacted under stirring for 3 hours at room temperature. The solvent is evaporated under reduced pressure and the residue is diluted in about 50 ml DMF. The mixture is left under stirring until the complete dissolution of the residue, then 1.15 g (8.9 mmol) L(-)α-amino-epsilon-caprolactam are added and the resulting mixture is heated to 60°C for 16 hours. The mixture is diluted with water and chloroform and after a short stirring the two phases are separated.

The organic phase is dried on sodium sulfate and evaporated under reduced pressure. The residue is diluted with a mixture chloroform/methanol in a volumetric ratio 98:2 and eluted on a silica gel column using as eluent a mixture chloroform/methanol with a variable volumetric ratio, starting from 100 % chloroform up to the final volumetric ratio 95:5.

0.8 g of the desired product are obtained
Melting point: 83-87 °C
¹HNMR: (200 MHz CDCl₃) 6.65-6.50 (m 2H); 6.40-6.30 (m 1H); 4.42 (dd 1H); 4.02 (s 1H); 3.25-2.95 (m 2H); 2.85 (s 3H); 2.80-2.60 (m 2H); 2.57 (s 3H); 2.00 -1.85 (m 2H); 1.75-1.10 (m 6H).
M.W. 371.46

| Elemental analysis | | |
|---|---|---|
| | Found | Calculated |
| C | 64.51 % | 64.67 % |
| H | 7.46 % | 7.33 % |
| N | 15.23 % | 15.08 % |

### Example 16

Preparation of eseroline L-prolylpyrrolidinamide (MGT2) Derivative of formula (I) wherein Y=NT'T''= 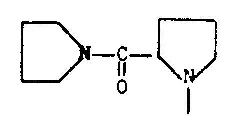
5.5 g (25 mmol) eseroline dissolved in 100 ml anhydrous THF are placed into a 250 ml flask fitted with a magnetic stirrer, a nitrogen inlet tube and a reflux condenser provided with a calcium chloride valve. The oxygen is eliminated by a continuous nitrogen flow for 45 minutes, then 5.6 g (35.6 mmol) N,N'-carbonyldiimidazole are added. The mixture is left to react under good stirring at room temperature for 3 hours, then 1.66 g (10 mmol) L-prolyl-N-pyrrolidinamide are added and the mixture is heated to 50 °C for 12 hours.

100 ml water are added and the solvent is evaporated under reduced pressure. The residue is extracted with ethyl acetate and the organic phase is washed with water.

The mixture is dried on sodium sulfate and brought to dryness. The product is purified by silica gel chromatography using as the eluent a mixture chloroform/methanol with a variable volumetric ratio as in Example 15.

1.2 g of the desired product are obtained.
¹HNMR: (200MHz CDCl₃) 6,65-6,50 (m 2H); 6,40-6,30 (m 1H); 4,02 (s 1H); 3,80-3,65 (m 1H); 3,60-3,25 (m 4H); 3,15 (m 1H); 2,90 (m 1H); 2,85 (s 3H); 2,80-2,60 (m 2H); 2,57 (s 3H); 2,20 -1,50 (m 8H); 1,42 (s 3H).
M.W. 400.52

| Elemental analysis | | |
|---|---|---|
| | Found | Calculated |
| C | 65.97 % | 66.12 % |
| H | 8.05 % | 8.27 % |
| N | 13.99 % | 13.75 % |

**Example 17** Preparation of eseroline N-ethanol-2-piperidone-3 aminocarbamate (MGT3) - Derivative of formula (I) wherein Y = L-NH- and wherein L = 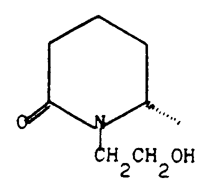
5.5 g (25 mmoles) eseroline dissolved in 100 ml methylene chloride are placed into a 250 ml flask fitted with a magnetic stirrer, a nitrogen inlet tube and a reflux condenser provided with a calcium chloride valve. The oxygen is eliminated by a continuous nitrogen flow for 45 minutes, then 4.9 g (30 mmol) N,N'-carbonyldiimidazole are added. The mixture is left to react under good stirring at room temperature for 3 hours, then the solvent is evaporated under reduced pressure and the residue is diluted with about 50 ml DMF. The obtained mixture is left under stirring until complete dissolution. Then 1.6 g (10 mmoles) N-ethanol-3 amino-valerolactam (10 mmol) are added and the mixture is heated to 50 °C for 16 hours. The reaction mixture is then diluted with water and chloroform and after a short stirring the two phases form. The organic phase is dried on sodium sulfate and evaporated to dryness under reduced pressure, the residue is diluted with methanol, filtered on carbon concentrated to a small volume and finally purified by flash chromatography on silica gel using as the eluent chloroform methanol as described in example 15.

0.6 g of the desired product are obtained.
¹HNMR: (200MHz CDCl₃) 6,65-6,50 (m 2H); 6,40-6,30 (m 1H); 4,02 (s 1H); 3,25-2,95 (m 2H); 2,85 (s 3H); 2,80 -2,60 (m 2H); 2,57 (s 3H); 2,00 -1,10 (m 4H);
M.W. 402,49

| Elemental analysis | | |
|---|---|---|
| | Found | Calculated |
| C | 62,84% | 62,67% |
| H | 7,66% | 7,51% |
| N | 13,87% | 13,92% |

**Example 18** - Preparation of eseroline N-D-glucosamine carbamate (MGT4)- Derivative of formula (I) wherein Y = L-NH- and L is D-glucosamine.

7.6 g (35 mmol) eseroline dissolved in 100 ml methylene chloride are placed into a 250 ml flask fitted with a magnetic stirrer, a nitrogen inlet tube and a reflux condenser provided with a calcium chloride valve. The oxygen is eliminated by a continuous nitrogen flow for 45 minutes, then 7.3 g (45 mmol) N,N'-carbonyldiimidazole are added. The mixture is left to react under good stirring at room temperature for 4 hours. The solvent is evaporated under reduced pressure and the residue is diluted in about 50 ml DMF . The obtained mixture is then left to stir until complete dissolution, then 1.8 g (10 mmol) D-glucosamine dissolved in DMF are added and the mixture is heated to 60 °C for 24 hours. The reaction mixture is then diluted with water and chloroform and then with n-butanol . The butanolic phase, washed with a NaCl saturated solution is then evaporated to dryness under reduced pressure at a lower temperature than 45°C . The residue is then crystallized from a mixture of ethanol/ethyl acetate 3 : 1.

0.4 g of the desired product are obtained.
¹HNMR: (200MHz DMSO d6) 7,00-6,85 (m 2H); 6,61 (d 1H); 4,10-3,9 (m 4H); 3,60-3,40 (m 6H); 2,95 (m 2H); 2,85 (dd 1H); 2,85 (dd 1H); 2,53 (s 3H); 2,00 -1,10 (s 3H);
M.W. 424,48

| Elemental Analysis | | |
|---|---|---|
| | Found | Calculated |
| C | 56,59% | 56,81% |
| H | 7,12% | 7,33% |
| N | 9,90% | 9,73% |

**Example 19** - Preparation of eseroline 2,6-dihydroxy-pyrimidine-4 carbamate (MGT5) derivative of the eseroline of formula (I) wherein Y = L-NH and L = 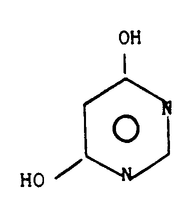
7.5 g (34 mmol) eseroline dissolved in 100 ml methylene chloride are placed into a 250 ml flask fitted with a magnetic stirrer, a nitrogen inlet tube and a reflux condenser provided with a calcium chloride valve. The oxygen is eliminated by a continuous nitrogen flow for 45 minutes, then 7.3 g (45 mmol) N,N'-carbonyldiimidazole are added. The mixture is left to react under good stirring at room temperature for some hours. The solvent is evaporated under reduced pressure and the residue is diluted in about 50 ml DMF. The obtained mixture is then left to stir until complete dissolution, then 1.3 g (10 mmol) 4-amino-2,6-dihydroxy-pyrimidine are added and the mixture is left to react at 65 °C for 24 hours. The reaction mixture is then diluted with water and chloroform and the two phases form. The aqueous phase is extracted with ethyl acetate and n-butanol, dried on sodium sulfate is evaporated to dryness under reduced pressure .

The residue is then diluted with a mixture of of acetonitrile and ethyl acetate and after hot filtration on carbon it is crystallized.

The desired product is thus obtained.
Melting point: 118-124°C
¹HNMR: (200MHz CDCl₃) 9,1(s 1H); 6,65 -6,50 (m, 2H); 6,40-6,30 (m 1H); 4,05 (s 1H); 2,85 (s 3H); 2,80-2,60 (m 2H); 2,55 (s 3H);
MW: 371,40

| Elemental Analysis | | |
|---|---|---|
| | Found | Calculated |
| C | 58,21% | 57,95% |
| H | 5,70% | 5,92% |
| N | 18,86% | 18,73% |

**Example 20** Preparation of eseroline 1,3 benzodioxol-5 carbamate (MGT7) - Derivative of formula (I) wherein Y = L-NH- and L = 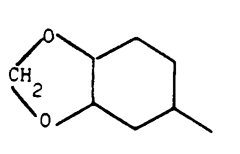
The eseroline (5.5 g; 25 mmol) is dissolved in a mixture of toluene and tetrahydrofuran and oxygen is accurately removed from the reaction environment by flowing nitrogen.

Then benzodioxol isocyanate (3.25 g; 20 mmol) dissolved in tetrahydrofuran and a catalytic amount of sodium (1mg), and the obtained mixture is heated to 50 °C under stirring for 12 hours.

The reaction mixture is then concentrated to dryness at a temperature < 40 °C, and the obtained residue is then dilutred with water and ethyl acetate.

The organic phase is then brought to dryness and purified by chromatography on silica gel, using as the eluent a mixture of chloroform/ methanol with variable volumetric ratios as described in example 15.

The desired product is then obtained as a white solid, which is then crystallized from ethyl acetate: hexane 3:1.
¹HNMR: (200MHz CDCl₃) 7,30-7,10(m 3H); 6,65 -6,50 (m, 2H); 6,40-6,30 (m 1H); 5,90 (d 2H); 4,05 (s 1H); 2,85 (s 3H); 2,80-2,60 (m 2H); 2,55 (s 3H);
M.W. 381,43

| Elemental Analysis | | |
|---|---|---|
| | Found | Calculated |
| C | 66,13% | 66,40% |
| H | 6,08% | 6,29% |
| N | 11,01% | 10,85% |

**Example 21** Preparation of eseroline-2-benzothiazol-carbamate (MGT8) -Derivative of formula (I) wherein Y =L-NH- and L= 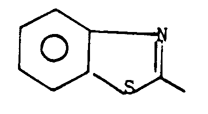
The eseroline (7.78,35 mmol) in methylenechloride is charged into a flask, and the oxygen is removed by flowing nitrogen.

N-N'-carbonyl-diimidazole (8.1 g, 50 mmol) and the reaction mixture is left under stirring for few hours.

The solvent is evaporated and the residue is diluted with dimethylformamide. The aminobenzothiazole (3,75 g; 25 mmol) dissolved in DMF is added and the obtained reaction mixture is heated to 60 °C for 16 hours.

The reaction mixture is cooled to room temperature and diluted with water and ethyl acetate. The organic phase, after being dried on sodium sulfate is evaporated to dryness and the obtained residue is purified by flash chromatography, by eluting with chloroform/methanol with variable volumetric ratios as described in example 15.

A white product is thus obtained.
¹HNMR: (200MHz CDCl₃) 7,90-7,20 (m 4H); 6,65 -6,55 (m, 2H); 6,40-6,30 (m 1H); 5,90 (d 2H); 4,00 (s 1H) 2,85 (s 3H); 2,80-2,60 (m 2H); 2,55 (s 3H);
M.W. 394,5

| Elemental analysis | | |
|---|---|---|
| | Found | Calculated |
| C | 63,94% | 64,11% |
| H | 5,62% | 5,77% |
| N | 14,20% | 14,49% |

### Example 22 -Preparation of MGT8 acid sulfate

The product (MGT8) may be treated with equimolar amounts of sulfuric acid in ethanol, successively the reaction mixture is evaporated to dryness and the product obtained is crystallized from acetonitrile/ethyl acetate 4:1. The spectral and elemental analyses confirm the assigned structure.

### Example 23 -Preparation of MGT1 sulfate

0,1 mol MGT1 obtained according to example 15 and 0.05 mol sulfuric acid are dissolved in absolute ethanol. The solvent is evaporated to dryness under reduced pressure from the obtained solution and the residue is crystallized from acetonitrile.

The spectral and elemental analyses confirm the assigned structure.

### Example 24 - Preparation of MGT2 acid tartrate

0.1 mol MGT2 are dissolved in absolute ethanol and treated with 0.1 mol tartaric acid dissolved in absolute ethanol, the obtained solution is evaporated to dryness under vacuum and the residue is crystallized from acetonitrile/ ethyl acetate 3:1.

The spectral and elemental analyses confirm the assigned structure.

### PHARMACOLOGIC ACTIVITY OF THE COMPOUNDS OD FORMULA (I)

### 1- TOXICOLOGIC AND PHARMACOLOGIC ACTIVITY OF THE COMPOUNDS OF FORMULA (I) WHEREIN THE SUBSTITUENT Y HAS THE MEANINGS GIVEN IN EACH OF THE ABOVE MENTIONED CLASSES A, B AND C

### - Electrocardiographic activity

Single doses of ESE001, ESE002, ESE003, ESE004, (in the range 0.4 - 7 mg/kg) determine a dose-dependence, reversible inhibition of the acetylcholinesterase activity, on the mouse brain.

The inhibitory activity of ESE001, ESE002, ESE003, ESE004, last longer than that of physostigmine.

Moreover ESE001, ESE002, ESE003, ESE004, even at higher doses, do not suppress the enzymatic activity.

### - Antagonist effect on the stimulating effect of scopolamine

At the dose of 1 mg/kg the scopolamine shows a clear stimulating effect (100 % increase in motor activity).

This is countered by the oral administration of 2.5 mg of ESE001, ESE002, ESE003, ESE004.

### - Pharmacokinetics

The kinetics of the four products to be considered, is studied in mouse after oral and intramuscular administration, of respectively 7 and 3.5 mg/kg respectively of ESE001, ESE002, ESE003, ESE004.

The maximum peak (30 mcg/ml) is found 1 hour after oral administration.

The plasma bioavailability (AUC os/ AUC im) is 30 % while the half-life is about 15 hours.

High concentrations are found in the brain, the brain bioactivity is 60 %.

A similar behaviour is observed in the heart.

The liver kinetics indicates that the compounds ESE001, ESE002, ESE003 ESE004, pass through into the urine unchanged.

### - Analgesic activity

The compounds of formula (I) wherein the substituent Y has the meanings given in each of the classes from A to C and in particular the compounds ESE001, ESE002, ESE003, ESE004, according to the present invention show also analgesic activity, for their ability to reduce pain.

This activity is demonstrated in the mouse with 2-phenylbenzoquinone test, which is a standard test for analgesic trials [Proc. Soc. Expte. Biol. Med. 95, 729, (1957).]

The procedure followed represents a modification of the test developed by Hoffner [Dtsch. Med. Wschr. 55, 731 (1929)].

### - Toxicity

LD50 is determined for the dose (mg/kg) at which 50 % of the animals died within 24 hours from the administration.

In the following table the results are reported of the toxicity trials relevant to the compounds of the invention.

**TABLE 1**

| Compounds | LD50 (mg/kg i.p.) |
|---|---|
| Physostigmine | 0.64 |
| ESE001 | 5 |
| ESE002 | >40 |
| | <80 |
| ESE003 | >40 |
| | <80 |
| ESE004 | >40 |
| | <80 |
| ESE005 | >40 |
| | <80 |
| ESE006 | >40 |
| | <80 |
| ESE007 | > 40 |
| | < 80 |
| ESE008 | > 30 |
| | < 60 |
| ESE009 | > 30 |
| | < 60 |
| ESE010 | > 30 |
| | < 60 |
| ESE011 | > 30 |
| | < 60 |
| ESE012 | > 30 |
| | < 60 |

### 2- TOXICOLOGIC AND PHARMACOLOGIC ACTIVITY OF THE COMPOUNDS OF FORMULA (I) WHEREIN THE SUBSTITUENT Y HAS THE MEANINGS GIVEN IN EACH OF THE ABOVE MENTIONED CLASSES D AND E

The compounds of formula (I) according to the present invention are useful in the treatment of several memory dysfunction characterized by a reduction of the cholinergic function.

The utility of these compounds resides in the ability to inhibit the cerebral acetylcholinesterase and to increase acetylcholine levels in the brain . The biological properties of some derivatives comprised in the definitione of formula (I) and wherein the substituent Y has the meanings given in each of the classes D and E were compared with those of physostigmine.

The acute toxicity (LD50), the "in vivo" cerebral and serous acetylcholinesterase inhibition, the spontaneous motility, the antagonism against the central stimulating action of an anticholinergic agent (scopolamine), the electroencephalographic activity, and the effect on the duration effect of curarization caused by succinylcholine.

Furthermore preliminary pharmacokinetic trials were carried out.

It resulted that the compounds according to the present invention in equimolar ratio with respect to physostigmine showed markedly higher therapeutic indices and a longer duration than those of physostigmine.

### Acute toxicity

The mouse was used (Swiss strain having the average weight of the male mice being 26 g and 22 g that of the female mice: 5+5 animals per dose, observation period: 7days)
The oral LD50 (suspension in 2% carboxymethylcellulose) were reported hereinbelow:

**Tab.2**

| LD50 in the mouse topo of physostigmine and of the compounds of formula (I) | |
|---|---|
| Physostigmine | 4,39 ± 1,14 mg/kg |
| MGT1 | 321,5 ± 3,21 mg/kg |
| MGT2 | 342,2 ± 11,16 mg/kg |
| MGT3 | 383,7 ± 41,2 mg/kg |
| MGT4 | 397,6 ± 39,72 mg/kg |
| MGT7 | 331,8 ± 23,94 mg/kg |

The compounds according to the present invention resulted to be from 70 to 90 times less toxic than physostigmine .

For all the substances the symptomathology of the toxic doses was essentially the same and was characterized by muscular tremors, depressive phenomena followed by death by asphyxia paralysis.

### "In vivo" anticholinesterase activity

The derivatives, under question and physostigmine suspended in 2% carboxymethylcellulose, were orally administered to groups of Wistar male rats (average weight about 250 g).

On the base of the obtained results in preliminary trials, the doses utilized for the comparison were comprised beween 0.2 and 0.4 mg/kg for physostigmine and between 1.5 and 4 mg/kg for the eseroline derivatives under question.

The groups of treated rats were sacrificed after 20, 60, and 120 minutes and the blood and the brain of each animal were immediately cooled to 0°C and maintained at this temperature during the successive treatments.

The acetylcholinesterase activity (AchE) was determined by using the Ellman et al's quick colorimetric method (Biochem. Pharmacol., 7, 88, 1961) and it was expressed as enzymatic inhibition percentage.

MGT1, MGT2, MGT3, MGT4, MGT7, in said tests proved to show an evident dose dipendent and reversible inhibitory activity of the acetylcholinesterase of the same magnitudo order as that of physostigmine, the effective doses for the compounds under question were an average from 4 to 7 times higher than that for physostigmine.

The duration of the antiacetylcholinesterase activity of the same compounds was considerably much longer than that of physostigmine.

### Spontaneous motility

The Dews' method was utilized, as modified according to Carminati (Arch. Int. Pharmacodyn. et Thér. 181, 68,1969).

MGT1, MGT2, MGT3, MGT4, MGT7 orally administered, caused in the mouse (SWISS strain of 22-24 g: 10 animals/group), starting from minimal doses of 3.5-5 mg/kg, a marked reduction of spontaneous motility (-39% on average):the same effect was shown by physostigmine at the dose of 0.6 mg/kg/os.

### Antagonist activity against the stimulating effect of an anticholinergic substance.

As the central anticholinergic stimulating agent the scopolamine was used subcutaneously injected to groups of 10 mice SWISS (22-26 8) at the dose of 1mg/kg.

At this dose the substance under question caused in all the animals a marked increase (80-100%) of the spontaneous motility, evaluated according to the above described method .

The preventive administration (30 minutes after) of the derivatives under question, at the doses of 2.5-3mg/kg/os, doses in themselves unactive on the motility, demonstrated to completely annul the excitatory effect of scopolamine.

Under the same experimental conditions, physostigmine showed the same antagonist effect at the dose of 0.4 mg/kg/os.

### Electroencephalographic activity

MGT1 was orally administered to a group of SWISS mice (28-30g).

Cortical electrodes were previously implanted onto the frontoparietal area to each of said animals according to the method described by Castellano and Oliverio (Brain Res.101, 317,1976).

The electrodes were connnected to a polygraph (Transamerica Instruments., model 8k42) in order to record the electroencephalogram.

The treatment with MGT1 at the dose of 5 mg/kg/os induced, in the course of the successive 30 minutes, the appearance of ecographical variations consisting of an amplitude increase and a slowing down electroencephalographic rate of the said variation were qualitatively the same as those observed as a consequence of the oral administration of 0.6 mg/kg physostigmine.

### Effect on the succinylcholine curarization duration

Physostigmine, at the i.v. dose of 0.1 mg administered to the anesthetized rabbit subjected to the artificial respiration, protracts considerably the duration of the succinyl choline curaric action, which can be detected by measuring the corneal reflex ( Marotta and Carminati, Arch. Int. Pharmacodyn, et Thér. 48, 255,1954).

By using the same method (Tawny Strain male rabbits of the average weight 2.2 kg under i.v.ethyl urethane anesthesia general conditions and subjected to artificial respiration), it was observed that the i.v. administration of 0.6 mg/kg MGT1 and MGT2 induced the same anticholinesterase effect with respect to the succinylcholine curarizing action as that caused by physostigmine.

### Pharmacokinetics

In some preliminary trials single doses of MGT1 equal to 2.5 and 5 mg/kg were orally administered to groups of Wistar rats (male rats of the average weight of 260 g: 5 per group).

The presence of the substance in the plasma was evidenced by HPLC. With this method, the identification threshold was 0.15 mcg per ml of plasma.

After the administration of 6.2 mg/kg/os, MGT1 resulted to be present at the concentration of 17.11 ± 3.81 mcg/ml, the maximum peak of 36.14 ± 7.12 mcg /ml was observed after 90'.

The values decreased then slowly (13,82 ± 4.12 mcg/ml after 12 hours) up to reaching the concentration 5.09 ± 1.71 mcg/ml after 24 hours.

The results obtained with the above reported tests demonstrate that the compounds according to the present invention and in particular those wherein the substituent Y has the meanings given in the class C and D are from 75 to 90 times less toxic than physostigmine and only 4-7 times less effective than physostigmine as anticholinesterase agents. Their therapeutic indices appear to be much more favourable than that of physostigmine. The duration of the anticholinesterase activity of the compounds according to the present invention results moreover higher than that of physostigmine.

## Claims

**1.** Eseroline derivatives of formula (I) 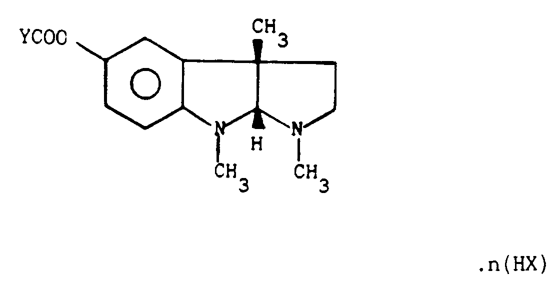 wherein Y is selected from the meanings given in each of the following classes:
A) -NH-NR₁R₂ wherein R₁ and R₂ form together a saturated, heterocyclic ring of from 3 to 6 carbon atoms, optionally containing another heteroatom selected from N,O,S, and such heterocyclic ring being optionally substituted at its carbon atoms or at its second heteroatom, in case this is a nitrogen atom, with groups selected from linear or branched C₁-C₁₀ alkyl radicals, optionally substituted on their turn with one or more hydroxy groups, or one or more carbon atoms of this heterocyclic ring optionally forming with one or more oxygen atoms, one or more 〉C=O groups;
B) -NH-R₃-T wherein R₃ is a C₁-C₁₀ linear or branched bivalent alkylene radical and T is a heterocyclic saturated radical containing from 3 to 4 carbon atoms and as heteroatoms one or more oxygen atoms;
C) 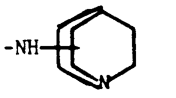
D) -NH-L wherein L is :
i) a saturated, unsaturated or an aromatic type heterocyclic ring, containing at least a nitrogen atom and having from 3 to 6 carbon atoms and optionally containing other heteroatoms selected from N, O,S, said heterocyclic ring being optionally condensed to one or more aromatic rings, and being optionally substituted at the carbon atoms with alkyl, aldehyde, ester, hydroxy groups, or halogen atoms, one or more of said carbon atoms of this heterocyclic ring optionally forming with oxygen atoms 〉C=O groups ,
and wherein the nitrogen atoms of L are optionally substituted with one or more alkylene-hydroxy, aldehyde, or alkylene-esters groups; or
ii) a saturated heterocyclic ring of from 1 to 2 oxygen atoms and of from 3 to 5 carbon atoms, being optionally condensed to one or more aromatic rings, or optionally substituted at the carbon atoms with one or more hydroxy groups or alkylene-hydroxy groups;
E) -NT'T'' wherein NT'T'' is a saturated heterocyclic ring of from 4 to 5 carbon atoms, substituted at the carbon atoms with one or more amidic groups, and said heterocyclic ring being optionally substituted at the remaining carbon atoms with one or more of hydroxy, alkylene-hydroxy and aldehyde groups; HX is a pharmaceutically acceptable organic or inorganic acid and n is the number of acid equivalents and is 0, 1/2, 1/3, 1 or 2.

**2.** The eseroline derivatives according to claim 1 wherein, when HX is an organic acid it is selected from tartaric acid, maleic acid, citric acid, when it is an inorganic acid, it is selected from hydrochloric acid sulfuric acid, phosphoric acid.

**3.** The eseroline derivatives according to claim 1 wherein when Y in has the meanings given in class A it is selected from:

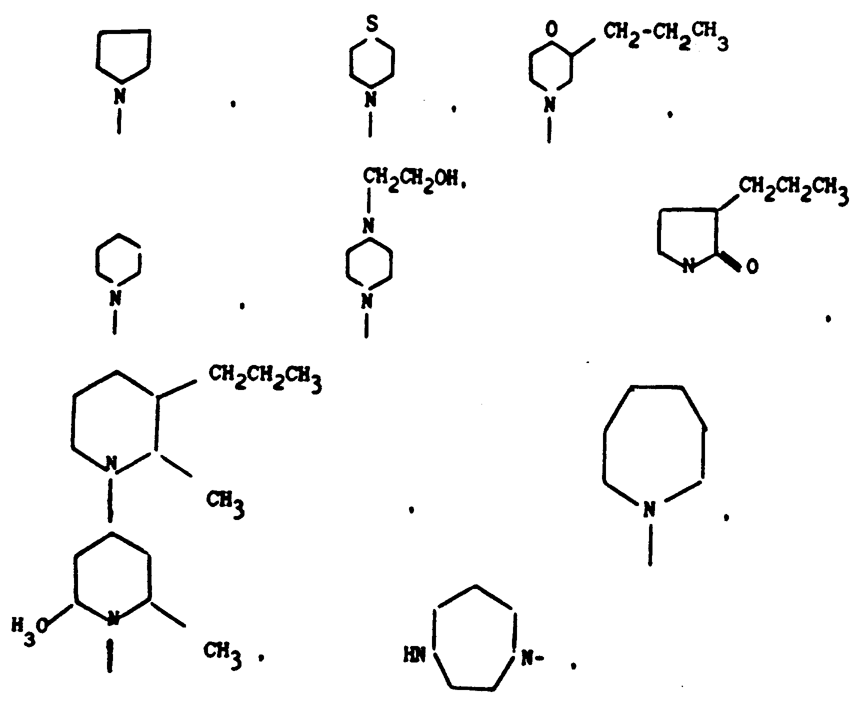

**4.** The eseroline derivatives according to claim 1 wherein when Y has the meanings given in class B, R₃ is -CH₂- and T is

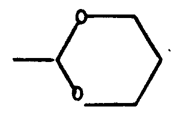

**5.** The eseroline derivatives according to claim 1 wherein when Y has the meanings given in class C it is:

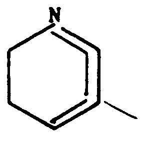

**6.** The eseroline derivatives according to claim 1 wherein when Y has the meaning given in class D , L has the meaning given in subclass (i) and it is : 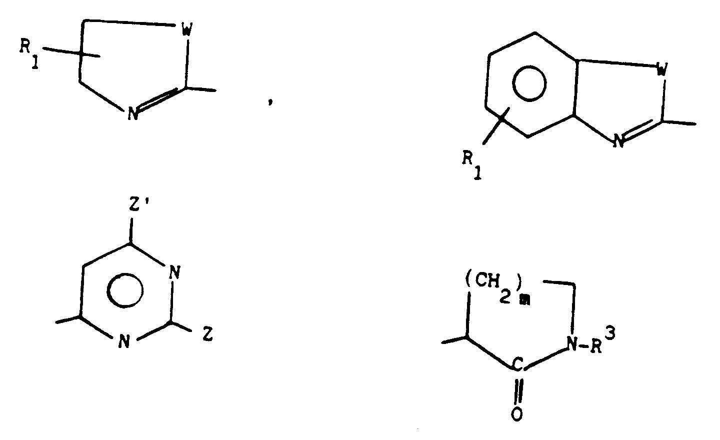 wherein W = N,O,S; Z and Z' equal or different from eachother are OH or Cl; R₁ is selected from H,CHO, COOR₂,a halogen atom,OH, C₁-C₅ alkyl, wherein R₂ is a C₁-C₅ alkyl, R₃ is selected from H, CH₂-CH₂OH, CHO, CH₂COOR₂ wherein R₂ has the above mentioned meanings, and m is = 1, 2 or 3.

**7.** The eseroline derivatives according to claim 1 wherein when Y has the meaning given in class D , L has the meaning given in subclass (ii) and it is : 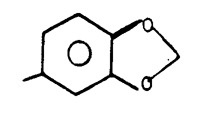 or glucosamine.

**8.** The eseroline derivatives according to claim 1 wherein when Y in the eseroline derivatives has the meanings given in class E (-NT'T'') it is : 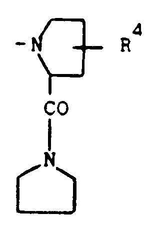 wherein R₄ is selected from: H, OH, CH₂OH, CHO.

**9.** A therapeutical composition containing as the active principle one or more eseroline derivatives of the formula (I), 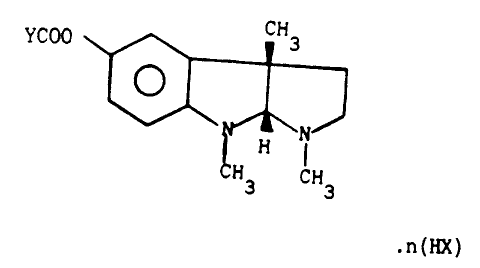 wherein Y is selected from the meanings given in each of the following classes:
A) -NH-NR₁R₂ wherein R₁ and R₂ form together a saturated, heterocyclic ring of from 3 to 6 carbon atoms, optionally containing another heteroatom selected from N,O,S, and such heterocyclic ring being optionally substituted at its carbon atoms or at its second heteroatom, in case this is a nitrogen atom, with groups selected from linear or branched C₁-C₁₀ alkyl radicals, optionally substituted on their turn with one or more hydroxy groups, or one or more carbon atoms of this heterocyclic ring optionally forming with one or more oxygen atoms, one or more 〉C=O groups;
B) -NH-R₃-T wherein R₃ is a C₁-C₁₀ linear or branched bivalent alkylene radical and T is a heterocyclic saturated radical containing from 3 to 4 carbon atoms and as heteroatoms one or more oxygen atoms;
C) 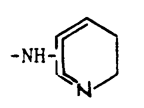
D) -NH-L wherein L is :
i) a saturated, unsaturated or an aromatic type heterocyclic ring, containing at least a nitrogen atom and having from 3 to 6 carbon atoms and optionally containing other heteroatoms selected from N, O,S, said heterocyclic ring being optionally condensed to one or more aromatic rings, and being optionally substituted at the carbon atoms with alkyl, aldehyde, esters, hydroxy groups, or halogen atoms, one or more of said carbon atoms of this heterocyclic ring optionally forming with oxygen atoms 〉C=O groups,
and wherein the nitrogen atom of L is optionally substituted with one or more alkylene-hydroxy, aldehyde, or alkylene-esters groups; or
ii) a saturated heterocyclic ring of from 1 to 2 oxygen atoms and of from 3 to 5 carbon atoms, being optionally condensed to one or more aromatic rings, or optionally substituted at the carbon atoms with one or more hydroxy groups or alkylene-hydroxy groups;
E) -NT'T'' wherein NT'T'' is a saturated heterocyclic ring of from 4 to 5 carbon atoms, substituted at the carbon atoms with one or more amidic groups, and said heterocyclic ring being optionally substituted at the remaining carbon atoms with one or more of hydroxy, alkylene-hydroxy and aldehyde groups;
HX is a pharmaceutically acceptable organic or inorganic acid and n is the number of acid equivalents and is 0, 1/2, 1/3, 1 or 2, in combination with suitable excipients and/or diluents.

**9.** The therapeutical composition according to claim 9 for the treatment of Alzheimer disease and for the treatment of myasthenia gravis, glaucoma and other diseases of neuromuscular origin.

**10.** The therapeutical composition according to claim 9 for the treatment of various memory dysfunctions, characterized by a cholinergic function reduction.

**11.** The therapeutical composition according to claim 8, having analgesic activity for the pain treatment.

**12.** The therapeutical compositions according to claim 9 wherein the active principle is contained in amounts ranging from 1 to 20 mg per unitary dose.

**13.** The therapeutical compositions according to claim 9 suitable to be orally and parenterally administered.

**14.** A process for preparing the eseroline derivatives of formula (I) 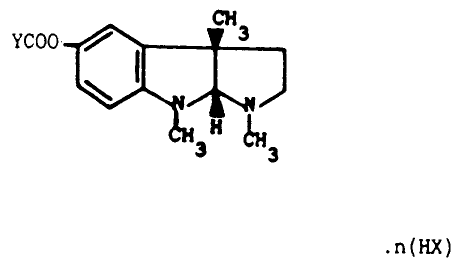 wherein Y is selected from the meanings given in each of the following classes:
A) -NH-NR₁R₂ wherein R₁ and R₂ form together a saturated, heterocyclic ring of from 3 to 6 carbon atoms, optionally containing another heteroatom selected from N,O,S, and such heterocyclic ring being optionally substituted at its carbon atoms or at its second heteroatom, in case this is a nitrogen atom, with groups selected from linear or branched C₁-C₁₀ alkyl radicals, optionally substituted on their turn with one or more hydroxy groups, or one or more carbon atoms of this heterocyclic ring optionally forming with one or more oxygen atoms, one or more 〉C=O groups;
B) -NH-R₃-T wherein R₃ is a C₁-C₁₀ linear or branched bivalent alkylene radical and T is a heterocyclic saturated radical containing from 3 to 4 carbon atoms and as heteroatoms one or more oxygen atoms;
C) 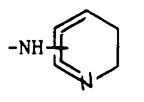
D) -NH-L wherein L is :
i) a saturated, unsaturated or an aromatic type heterocyclic ring,
containing at least a nitrogen atom and having from 3 to 6 carbon atoms and optionally containing a other heteroatoms selected from N, O,S, said heterocyclic ring being optionally condensed to one or more aromatic rings, and being optionally substituted at the carbon atoms with alkyl, aldehyde, esters, hydroxy groups, or halogen atoms, one or more of said carbon atoms of this heterocyclic ring optionally forming with oxygen atoms 〉C=O groups ,
and wherein the nitrogen atoms of L are optionally substituted with one or more alkylene-hydroxy, aldehyde, or alkylene-esters groups; or
ii) a saturated heterocyclic ring of from 1 to 2 oxygen atoms and of from 3 to 5 carbon atoms, being optionally condensed to one or more aromatic rings, or optionally substituted at the carbon atoms with one or more hydroxy groups or alkylene-hydroxy groups,
HX is a pharmaceutically acceptable organic or inorganic acid and n is the number of acid equivalents and is 0, 1/2, 1/3, 1 or 2; comprising the following steps:
a) reacting the eseroline of formula (II): 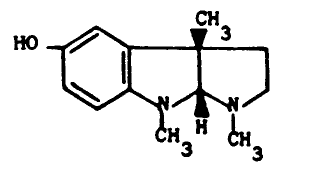 with the isocyanate of formula (III):
G-N=C=O (III)
wherein G has the following meanings :
A') it is NR'₁R₂' wherein NR'₁R'₂ has the same meaning of -NR₁R₁R₂ with the only differences that when the heterocyclic ring contains a second nitrogen atom, this has been previously protected with a suitable protecting group and /or in the case the heterocyclic ring brings one or more substituents consisting of alkylenehydroxy , these have been previously protected with suitable protecting groups;
B') it is -R₃-T, wherein R₃ and T have the above mentioned meanings;
C') it is 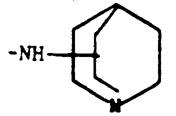
D') it is L', wherein L' has the same meaning of L, with the only differences that when the heterocyclic ring contains one or more - -NH- groups in its structure , these have been previously protected with a suitable protecting group, and/or in case the heterocyclic ring is substituted at the carbon atoms with alkylene-hydroxy or hydroxy groups, and/or at the nitrogen atoms with alkylene-hydroxy groups , these substituents have been previously protected with suitable protecting groups,
thereby obtaining the intermediate (IV) 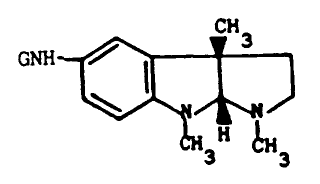
b) removing the above mentioned protecting groups, in case these are present, thereby obtaining the compounds of formula (I) having n=0;
c) reacting the intermediate obtained in step (a) or (b) with an equivalent amount of the pharmaceutically acceptable acid HX in an alcoholic solvent, thereby obtaining the eseroline derivative of formula (I) having n different from 0.

**15.** A process for preparing the eseroline derivatives of formula (I) 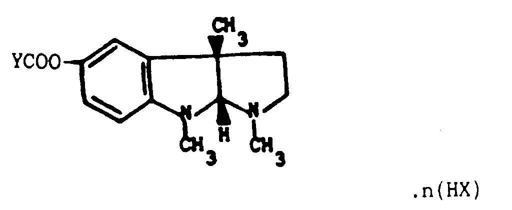 wherein Y is selected from the meanings given in each of the following classes:
A) -NH-NR₁R₂ wherein R₁ and R₂ form together a saturated, heterocyclic ring of from 3 to 6 carbon atoms, optionally containing another heteroatom selected from N,O,S, and such heterocyclic ring being optionally substituted at its carbon atoms or at its second heteroatom, in case this is a nitrogen atom, with groups selected from linear or branched C₁-C₁₀ alkyl radicals, optionally substituted on their turn with one or more hydroxy groups, or one or more carbon atoms of this heterocyclic ring optionally forming with one or more oxygen atoms, one or more 〉C=O groups;
B) -NH-R₃-T wherein R₃ is a C₁-C₁₀ linear or branched bivalent alkylene radical and T is a heterocyclic saturated radical containing from 3 to 4 carbon atoms and as heteroatoms one or more oxygen atoms;
C) 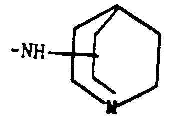
D) -NH-L wherein L is :
i) a saturated, unsaturated or an aromatic type heterocyclic ring, containing at least a nitrogen atom and having from 3 to 6 carbon atoms and optionally containing other heteroatoms selected from N, O,S, said heterocyclic ring being optionally condensed to one or more aromatic rings, and being optionally substituted at the carbon atoms with alkyl, aldehyde, esters, hydroxy groups, or halogen atoms, one or more of said carbon atoms of this heterocyclic ring optionally forming with oxygen atoms 〉C=O groups,
and wherein the nitrogen atoms of L are optionally substituted with one or more alkylene-hydroxy, aldehyde, or alkylene-esters groups; or
ii) a saturated heterocyclic ring of from 1 to 2 oxygen atoms and of from 3 to 5 carbon atoms, being optionally condensed to one or more aromatic rings, or optionally substituted at the carbon atoms with one or more hydroxy groups or alkylene-hydroxy groups;
E) -NT'T'' wherein NT'T'' is a saturated heterocyclic ring of from 4 to 5 carbon atoms, substituted at the carbon atoms with one or more amidic groups, and said heterocyclic ring being optionally substituted at the remaining carbon atoms with one or more of hydroxy, alkylene-hydroxy and aldehyde groups;
HX a pharmaceutically acceptable organic or inorganic acid and n is the number of acid equivalents and is 0, 1/2, 1/3, 1 or 2, comprising the following steps:
a') reacting the eseroline of formula (II) under nitrogen atmosphere in an ethereal or in a halo-hydrocarbon solvent at room temperature, with the compound of formula (V):
MCOM' (V)
wherein M and M' have the following meanings :
M = M'= imidazole;
M = M' = 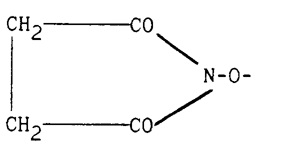 M = 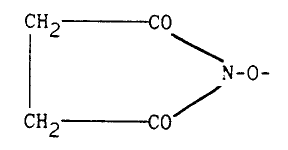
M'= -O-CH₂-C(Cl)₃;
thereby obtaining the intermediate (VI) 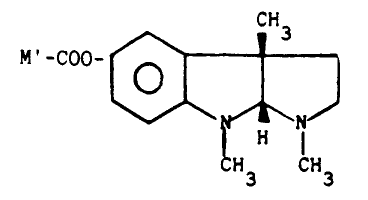
b') reacting the intermediate (VI) obtained in step (a) with the amine of formula (VII) :
YH (VII)
at a temperature comprised between -10 and 100°C in the same solvent used in step (a) in case the compund YH is soluble in this solvent otherwise reacting the above mentioned intermediate (VI) previously isolated from the reaction mixture of step (a) under the above cited operating conditions and with the above defined reactant (VII) in a dipolar aprotic solvent, thereby obtaining the eseroline derivative of formula (I) having n= 0;
c') treating the intermediate obtained in step (b) in an alcoholic solvent with equivalent amounts of the pharamceutically acceptable acid HX, thereby obtaining the eseroline derivatives of formula (I), having n different from 0.
